# EUROPÄISCHE PATENTSCHRIFT

(11) **EP 0 442 063 B1**
(45) Veröffentlichungstag und Bekanntmachung des Hinweises auf die Patenterteilung: **18.08.1993**
(21) Anmeldenummer: 90123208.2
(22) Anmeldetag: 04.12.1990
(51) Int. Cl.: A61K 31/40

(54) **Verwendung von 2',4',6'-Trimethoxy-4-(1-pyrrolidinyl)butyrophenon zur lokalen Behandlung von trophischen Erkrankungen**
Use of 2',4',6'-trimethoxy-4-(1-pyrrolidinyl) butyrophenone for the topical treatment of trophic diseases
Emploi du 2',4',6'-triméthoxy-4-(1-pyrrolidinyl) butyrophénone dans le traitement topique de maladies trophiques

(30) Priorität: 10.02.1990 DE 4004152; 27.11.1990 DE 4037658
(43) Veröffentlichungstag der Anmeldung: 21.08.1991
(73) Patentinhaber: A. Nattermann & Cie. GmbH, D-50829 Köln (DE)
(72) Erfinder: Viehmann, Wolfgang, Dr., W-8930 Schwabmünchen (DE); Högn, Thomas, Dr., W-5000 Köln 40 (DE)
(74) Vertreter: Döring, Wolfgang, Dr.-Ing. Patentanwälte Hauck, Graalfs, Wehnert, Döring, Siemons

(56) Entgegenhaltungen:
- EP-A- 0 369 105
- SOINS, Nr. 537, Juni 1990, Seiten 57-58; C. GADENNE-TESSE: "Buflomédil en application locale. Intéret dans le traitement des pertes de substance cutanée"
- FORTSCHR. MED., Band 103, Nr. 1-2, 1985, Seiten 51-55; B. FAGRELL et al.: "Wirking von Buflomedil auf die Mikrozirkulation der Haut bei Akralgangrän"
- Z. ALLG. MED., Band 61, Nr. 1-2, 1985, Seiten 32-35, Hippokrates Verlag GmbH, Stuttgart, DE; H. PETRY et al.: "Claudicatio intermittens und trophische Hautstörungen"
- DIALOG INFORMATION SERVICE, file 5 (BIOSIS), accession no. 7159603; E. BERNARDINI et al.: "Venous retrograde pulse therapy in infected statis ulcers", & FOLHA MED. 98(4), 1989, Seiten 247-251

## Beschreibung

Die vorliegende Erfindung betrifft eine neue Verwendung von 2',4',6'-Trimethoxy-4-(1-pyrrolidinyl) butyrophenon, seinen Salzen und/oder seinen Derivaten zur Herstellung eines pharmazeutischen Produktes.

Es ist bekannt, 2',4',6'-Trimethoxy-4-(1-pyrrolidinyl) butyrophenon in Form seines Hydrochlorids als Dragee oder Injektionslösung zur Behandlung von peripheren arteriellen Durchblutungsstörungen und diabetischen Angiopathien zu verwenden. Hierbei weist diese Verbindung, die auch als Buflomedil bezeichnet wird, die nachfolgende Strukturformel auf:

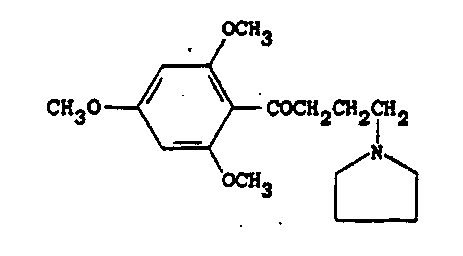

Trophische Störungen sind ernährungsbedingte und/oder wachstumsbedingte Störungen des Gewebes oder der Organe. Die hierbei auftretenden Beschwerden, wie etwa Stauungsdermatosen oder Ulcus cruris venosum, sind dabei die schwersten Folgen einer chronischen venösen Insuffizienz. Diese werden durch eine konstitutionelle, anlagenbedingte Venenwandschwäche oder als Folge thrombotischer Erkrankungen hervorgerufen. Ebenfalls kann man die Dekubitalulcera zu den trophischen Hautstörungen zählen, wobei man die Dekubitalulceraerkrankungen auf regionale Durchblutungsstörungen, die besonders oft bei immobilisierten bettlägerigen Patienten auftreten, zurückführen kann. Hierbei wird diese Erkrankung noch durch Benetzung der entsprechenden Hautflächen mit Harn oder Schweiß gefördert.

So zeigt eine Untersuchung in Pharmakinetik 8 (6), 1986, S. 21 bis 24, daß etwa 2% der Bevölkerung in den westlichen Industriestaaten Ulcera im Bereich der Beine aufweisen, wobei hiervon rd. 90% venöse Ursachen haben.

Eine medikamentöse Standardtherapie für die zuvor genannten und beschriebenen Erkrankungen, Störungen bzw. Beschwerden, insbesondere auch für die Behandlung von Ulcus cruris venosum, Dekubitalulcera oder Stauungsdermatosen, existiert bisher nicht. Es erfolgt lediglich eine symptomatische Behandlung, ohne daß hieraus in vielen Fällen ein eindeutige Heilung resultiert.

Nach der klassischen Schulmedizin wird der Ulcus gereinigt und gleichzeitig antimikrobiell und antiphlogistisch behandelt. Die Reinigung des Ulcus kann dabei auf mechanischem, osmotischem oder enzymatischem Wege erfolgen. Zusätzlich können dabei granulationsfördernde und epithelisierende Mittel eingesetzt werden. Durch ein Anlegen von Druckverbänden soll der venöse Abfluß auf physikalischem Wege erleichtert werden, wobei eine Ruhigstellung des Patienten vorgesehen ist, wie dies der Publikation von Mörl, Fortschr. Med. 104 (21), 1986 zu entnehmen ist.

Überraschenderweise ist nun gefunden worden, daß die zuvor beschriebenen trophischen Erkrankungen, der hierauf zurückzuführende Folgeerkrankungen sowie der entsprechenden Beschwerden, insbesondere Stauungsdermatosen, Ulcus cruris venosum und/oder Dekubitalulcera dann mit Erfolg behandelt werden können, wenn eine pharmazeutische Zubereitung lokal auf die erkrankten Bereiche verwendet wird, die als Wirkstoff bzw. als Wirkstoffkombination 2',4',6'-Trimethoxy-4-(1-pyrrolidinyl) butyrophenon, Salze und/oder Derivate davon enthält. Hierbei stellte man fest, daß bereits nach einer kurzen topischen Anwendung einer derartigen pharmazeutischen Zubereitung eine dauerhafte Heilung dieser Erkrankungen bzw. Beschwerden auftrat. Insbesondere bei nassen oder nässenden offenen Wunden bei Ulceration trockneten diese nach wenigen topischen Behandlungen mit dem zuvor genannten Arzneimittel ab, selbst wenn das entsprechende Arzneimittel in einer flüssigen Darreichungsform verwendet wurde.

So konnte beobachtet werden, daß bereits nach kurzer Zeit, bezogen auf die sonst übliche Therapiedauer, Gefäßeinsprossungen aus der Tiefe des Ulcus sowie deutlich erkennbare Granulationsinseln auftraten. Ebenso erfolgte die entsprechende Heilung sowohl aus der Tiefe des Ulcus wie vom Rand her durch Gewebezuwachs, so daß in der Regel entsprechende kosmetische Nachbehandlungen oder Korrekturen der ursprünglich erkrankten Bereiche entfallen konnten. Ferner konnte die Anwendung von Antibiotika, Sterilisations- und Reinigungsmitteln, die bei der klassischen Therapie zur Bekämpfung der zusätzlich auftretenden bakteriellen Infektion erforderlich sind, deutlich reduziert werden.

Besonders schnelle und für den Patienten schmerzlindernde Therapieerfolge werden erzielt, wenn das verwendete Arzneimittel, das 2',4',6'-Trimethoxy-4-(1-pyrrolidinyl) butyrophenon, Salze und/oder Derivate davon als Wirkstoffe enthält, lokal auf die jeweils erkrankten Bereiche aufgetragen wird. Hierbei kann dieses Arzneimittel als flüssiges, halbfestes oder festes Arzneimittel eingesetzt werden.

Flüssige Arzneimmittel stellen dabei Lösungen, Suspensionen, Emulsionen oder Dispersionen der zuvor genannten Wirkstoffe bzw. Wirkstoffkombinationen in Form von Tropfen, Tinkturen und Sprays dar. Als halbfeste Arzneimittel kommen beispielsweise Gele, Salben, Cremes und Schäume infrage, während unter festen Arzneimitteln beispielsweise Pulver, Puder, Granulate, Pellets und Mikrokapseln fallen. Ausgeschlossen sind jedoch die Zusammensetzungen, die in EP-A-0 369 105 im Sinne des Art. 54(3) EPÜ beschrieben sind.

Wird das pharmazeutische Produkt, das als Wirkstoff 2',4',6'-Trimethoxy-4-(1-pyrrolidinyl) butyrophenon, Salze und/oder Derivate davon enthält, in Form einer flüssigen Darreichungsform verwendet, so empfiehlt es sich, möglichst reizfreie Verdünnungsmittel, wie beispielsweise Wasser, einwertige Alkohole, insbesondere Ethanol, mehrwertige Alkohole, insbesondere Glycerin und/oder Propandiol, Polyglykole, insbesondere Polyethylenglykole und/oder Miglyol, Glycerinformal, Dimethylisosorbid, natürliche und synthetische Öle und/oder Ester auszuwählen.

Für die Herstellung von halbfesten Darreichungsformen, wie beispielsweise Gele, Salben, Cremes und Schäume, eignen sich neben den vorgenannten Verdünnungsmitteln zusätzlich noch Grundmassen, wie beispielsweise Bentonit, Veegum, Guarmehl und/oder Cellulosederivate, insbesondere Methylcellulose und/oder Carboxymethylcellulose. Ebenso kommen als Grundmassen anstelle der zuvor genannten Grundmassen oder zusätzlich zu den zuvor genannten Grundmassen Polymere aus Vinylalkohol und Vinylpyrrolidon, Alginate, Pektine, Polyacrylate, feste und/oder flüssige Polyethylenglykole, Paraffine, Fettalkohole, Vaseline und/oder Wachse, Fettsäuren und/oder Fettsäureester in Frage. Für die Herstellung von festen Darreichungsformen, wie beispielsweise Pulver, Puder, Granulate, Pellets und Mikrokapseln, besteht die Möglichkeit, die zuvor genannten Wirkstoffe ohne Streckmittel einzusetzen. Dieses Arzneimittel eignet sich insbesondere zur Behandlung von solchen der vorstehend beschriebenen Erkrankungen, die schon sehr weit fortgeschritten sind, so daß eine erhöhte Konzentration an Wirkstoffen zunächst erforderlich ist. Bei weniger schlimmen Krankheitszuständen oder mit fortschreitender Heilung der Krankheit kommen solche Ausführungsformen der festen pharmazeutischen Zubereitung zur Anwendung, die Streckmittel, wie beispielsweise koloidale Kieselsäure, Talkum, Milchzucker, Stärkepulver, Zucker, Cellulosederivate, Gelatine, Metalloxyde und/oder Metallsalze enthalten, wobei hier die Konzentration des Wirkstoffes bzw. der Wirkstoffkombination zwischen 0,001 Gew.% und 50 Gew.% variiert.

Darüber hinaus können die zuvor beschriebenen Ausführungsformen des pharmazeutischen Produktes wahlweise je nach Darreichungsform noch weitere Bestandteile, wie beispielsweise Konservierungsmittel, Stabilisatoren, Tenside, Emulgatoren, Penetrationsförderer, Spreitungsmittel und/oder Treibmittel enthalten.

Besonders bevorzugt werden bei der Herstellung des pharmazeutischen Produktes solche Zusatzstoffe, die aufgrund ihrer Stoffeigenschaften Autosterilität garantieren, eingesetzt, so daß dann auf eine zusätzliche Konservierung des pharmazeutischen Produktes verzichtet werden kann. Hierfür besonders geeignet sind solche Zusätze, die Propylenglykol und/oder Glycerin enthalten, wobei diese speziellen Zusätze vorzugsweise dann mehr als 10 Gew.% des pharmazeutischen Produktes ausmachen. Hierbei weisen diese Zusätze dann noch den Vorteil auf, daß sie eine gute Verträglichkeit, insbesondere auch Hautver-träglichkeit, besitzen.

Besonders geeignet ist es, wenn das so hergestellte pharmazeutische Produkt einen pH-Wert aufweist, der in einem Bereich zwischen 3,5 bis 8, vorzugsweise 4,5 bis 6,5, liegt. Dies kann beispielsweise dadurch erreicht werden, daß bei der Herstellung des pharmazeutischen Produktes geeignete Puffersubstanzen zugegeben werden. Bei flüssigen oder halbfesten pharmazeutischen Produkten ist es ebenso möglich, hier bei ihrer Herstellung über geeignete Säuren bzw. Basen die zuvor genannten pH-Werte einzustellen.

Weiterhin kann es bei der Herstellung des pharmazeutischen Produktes erforderlich sein, daß dem pharmazeutischen Produkt zusätzlich noch Antibiotika und/oder Sterilisationsmittel zugesetzt werden. Hierbei variiert die Konzentration der Antibiotika in einem Bereich zwischen 5 mg und 300 mg, insbesondere zwischen 50 mg und 200 mg, jeweils bezogen auf 1 g fertiges pharmazeutisches Produkt. Der Zusatz dieser Antibiotika bzw. Sterilisationsmittel beschleunigt einerseits die Heilung dadurch, daß eine Sekundärinfizierung der zu behandelnden erkrankten Flächen verhindert wird. Weiterhin stellen diese Antibiotika bzw. Sterilisationsmittel sicher, daß bei der Herstellung, beim Abfüllen bzw. beim Verpacken eine Keimfreiheit gewährleistet ist, so daß ein derartiges pharmazeutisches Produkt, insbesondere in seiner flüssigen und halbfesten Darreichungsform, weniger als 1000, vorzugsweise weniger als 100 vermehrungsfähiger, nicht pathogener Mikroorganismen pro g enthält.

Als Wirkstoff wird für die Herstellung des pharmazeutischen Produktes das eingangs strukturformelmäßig wiedergegebene 2',4',6'-Trimethoxy-4-(1-pyrrolidinyl) butyrophenon eingesetzt. Ebenso können anstelle des 2',4',6'-Trimethoxy-4-(1-pyrrolidinyl) butyrophenon oder zusätzlich hierzu Salze, insbesondere das entsprechende Hydrochlorid, Maleat und/oder Alkali und/oder Erdalkalisalze des am Aromaten und/oder am Pyridin sulfatierten und/oder sulfonierten 2',4',6'-Trimethoxy-4-(1-pyrrolidinyl) butyrophenon, bei der Herstellung des pharmazeutischen Produktes eingesetzt werden. Auch haben sich solche Ausführungsformen des pharmazeutischen Produktes zur Behandlung der vorstehend genannten Krankheiten bewährt, die zusätzlich zu den zuvor aufgeführten Wirkstoffen oder anstelle der zuvor genannten Wirkstoffe Derivate, vorzugsweise pharmakologisch aktive Stoffwechselprodukte (Metabolite) des 2',4',6'-Trimethoxy-4-(1-pyrrolidinyl) butyrophenon, insbesondere das p-Desmethyl-2',4',6'-Trimethoxy-4-(1-pyrrolidinyl) butyrophenon, aufweisen.

Eine besondere geeignete Darreichungsform des vorstehend ausführlich beschriebenen pharmazeutischen Produktes sieht vor, daß das feste, flüssige oder halbfeste pharmazeutische Produkt auf einen Gazestreifen, eine Kompresse oder ein Pflaster gegeben wird, so daß ein derartiger Gazestreifen, eine derartige Kompresse bzw. ein derartiges Pflaster lokal auf die jeweils zu behandelnde Stelle aufgetragen wird.

Wie bereits bei der festen Darreichungsform des pharmazeutischen Produktes beschrieben wurde, richtet sich bei der Herstellung des pharmazeutischen Produktes die Konzentration der Wirkstoffe nach der jeweiligen Schwere der Erkrankung. Üblicherweise variiert die Wirkstoffkonzentration bei festen Arzneimittelzusammensetzungen zwischen 0,001 Gew.% und 100 Gew.% und bei flüssigen und halbfesten Arzneimittelzusammensetzungen zwischen 0,01 Gew.% und 100 Gew.%, vorzugsweise zwischen 1 Gew.% und 50 Gew.%. Die Differenz zu 100 Gew.% ergibt sich dann aus dem jeweiligen Anteil der zuvor genannten Verdünnungsmittel bzw. weiteren Zusätzen.

Die Abfüllung eines derartigen pharmazeutischen Produktes erfolgt dann in den an sich bekannten Behältnissen, wie Flaschen, Tuben, Puder- und Streudosen sowie Siegelrandbeuteln, die ggf. mit Dosierungshilfen, wie Tropfenbildeeinrichtungen, Dosierventilen oder Dosierkammern, ausgestattet sind.

Die erfindungsgemäße Verwendung wird nachfolgend anhand von zwei Fallbeispielen näher beschrieben.

### Beispiel 1

Eine 50 jährige Patientin, die seit mehreren Jahren an einem Ulcus cruris venosum litt, galt nach Behandlung mittels gängiger Therapieprinzipien als klinisch austherapiert. Die Wunde mit einer Größe von 0,4 cm näßte und juckte, verbreitete einen üblen Geruch und war von schmierigen gelblichen Belägen bedeckt.
Zur Behandlung wurde im täglichen Wechsel die Wunde mit Gentiane violett ausgepustet bzw. es wurde ein mit 2',4',6'-Trimethoxy-4-(1-pyrrolidinyl) butyrophenon-Hydrochloridlösung getränkter Gazestreifen direkt auf die Wunde gelegt. Die Konzentration der Lösung betrug 100 mg 2',4',6'-Trimethoxy-4-(1-pyrrolidinyl) butyrophenon-Hydrochlorid in 10 ml Natriumchlorid-Lösung. Nach 3 Wochen konnte die Patientin mit einem ausgeheilten Ulcus cruris aus der Klinik entlassen werden.

### Beispiel 2

Eine als medizinisch austherapiert geltende Patientin (56 Jahre), seit mehreren Jahren an mehreren Ulcera cruris (0,2 bis 6 cm) am linken Fuß bzw. Unterschenkel leidend, wurde folgendermaßen behandelt:
Anfangs wurden auf die gesäuberten Wunden täglich ein mit 2',4',6'-Trimethoxy-4-(1-pyrrolidinyl) butyrophenon Hydrochloridlösung (100 mg 2',4',6'-Trimethoxy-4-(1-pyrrolidinyl) butyrophenon-Hydrochlorid in 10 ml Natriumchlorid-Lösung) getränkter Gazestreifen direkt aufgelegt. Nach beginnender Gefäßeinsprossung wurde die Therapie in zweitägigem Rhythmus weitergeführt. Nach 3 Monaten Therapiedauer waren die Ulcera stark verkleinert. Ein deutlich deckender Gewebezuwachs war aus der Tiefe erkennbar.

## Patentansprüche

1. Verwendung von 2',4',6'-Trimethoxy-4-(1-pyrrolidinyl) butyrophenon, seinen Salzen und/oder seinen Derivaten zur Herstellung eines pharmazeutischen Produktes für die topische Behandlung von trophischen Erkrankungen, der hierauf zurückzuführenden Folgeerkrankungen, Stauungsdermatosen, Ulcus cruris, Ulcus cruris venosum und/oder Dekubitalulcera, wobei das pharmazeutische Produkt lokal auf die jeweils erkrankten Bereiche aufgetragen wird.

2. Verwendung nach Anspruch 1, dadurch gekennzeichnet, daß das pharmazeutische Produkt einen pH-Wert zwischen 3,5 und 8, vorzugsweise 4,5 und 6,5, besitzt.

3. Verwendung nach Anspruch 1 oder 2, dadurch gekennzeichnet, daß das pharmazeutische Produkt ferner Antibiotika und/oder Sterilisationsmittel aufweist.

4. Verwendung nach Anspruch 3, dadurch gekennzeichnet, daß das pharmazeutische Produkt Antibiotika in einer Konzentration zwischen 5 mg und 300 mg, insbesondere 50 mg und 200 mg, pro g pharmazeutisches Produkt aufweist.

5. Verwendung nach Anspruch 3 oder 4, dadurch gekennzeichnet, daß das pharmazeutische Produkt als Sterilisationsmittel mindestens 10 Gew.% Propylenglykol und/oder mindestens 10 Gew.% Glycerin enthält.

6. Verwendung nach einem der vorangehenden Ansprüche, dadurch gekennzeichnet, daß das pharmazeutische Produkt das 2',4',6'-Trimethoxy-4-(1-pyrrolidinyl)butyrophenon, die entsprechenden Salze und/oder die Derivate davon in einer Konzentration zwischen 1 Gew.% und 50 Gew.% aufweist.

7. Verwendung nach einem der vorangehenden Ansprüche, dadurch gekennzeichnet, daß das pharmazeutische Produkt p-Desmethyl-2',4',6'-Trimethoxy-4-(1-pyrrolidinyl)butyrophenon enthält.

## Claims

1. The use of 2',4',6'-trimethoxy-4-(1-pyrrolidinyl) butyrophenone, its salts and/or its derivatives for the manufacture of a pharmaceutical product for the topical treatment of trophical diseases, the diseases resulting herefrom, obstruction dermatoses, ulcus cruris, ulcus cruris venosum and/or decubital ulcera, characterized in that the pharmaceutical product is applied locally.

2. The use according to claim 1, characterized in that the pharmaceutical product has a pH of between 3.5 and 8.5, preferably of between 4.5 and 6.5.

3. The use according to claim 1 or 2, characterized in that the pharmaceutical product further includes antibiotics and/or sterilization agents.

4. The use according to claim 3, characterized in that the pharmaceutical product contains antibiotics in a concentration of between 5 mg and 300 mg, especially of between 50 mg and 200 mg, per g of the pharmaceutical product.

5. The use according to claim 3 or 4, characterized in that the pharmaceutical product contains as sterilization agent at least 10 % by weight propylene glycol and/or at least 10 % by weight glycerol.

6. The use according to one of the preceding claims, characterized in that the pharmaceutical product contains the 2',4',6'-trimethoxy-4-(1-pyrrolidinyl) butyrophenone, the corresponding salts and/or the derivatives thereof in a concentration of between 1 % by weight and 50 % by weight.

7. The use according to one of the preceding claims, characterized in that the pharmaceutical product contains p-desmethyl-2',4',6'-trimethoxy-4-(1-pyrrolidinyl) butyrophenone.

## Revendications

1. Utilisation de la 2',4',6'-triméthoxy-4-(1-pyrrolidinyl)butyrophénone, de ses sels et/ou de ses dérivés en vue de la préparation d'un produit pharmaceutique destiné au traitement topique de maladies trophiques, de séquelles ou de maladies secondaires qui doivent leur être attribuées, de dermatoses congestives, les ulcères cruraux, les ulcères cruraux veineux ou variqueux et/ou d'escarres de décubitus ou décubitus aigus, conformément à laquelle on applique le produit pharmaceutique localement sur les zones touchées par la maladie.

2. Utilisation suivant la revendication 1, caractérisée en ce que le produit pharmaceutique possède une valeur de pH qui varie de 3,5 à 8, de préférence de 4,5 à 6,5.

3. Utilisation suivant la revendication 1 ou 2, caractérisée en ce que le produit pharmaceutique contient en outre des antibiotiques et/ou des agents de stérilisation.

4. Utilisation suivant la revendication 3, caractérisée en ce que le produit pharmaceutique contient des antibiotiques en une concentration qui varie de 5 mg à 300 mg, plus particulièrement de 50 mg à 200 mg, par gramme de produit pharmaceutique.

5. Utilisation suivant la revendication 3 ou 4, caractérisée en ce que le produit pharmaceutique contient, à titre d'agents de stabilisation, au moins 10% en poids de propylèneglycol et au moins 10% en poids de glycérine.

6. Utilisation suivant l'une quelconque des revendications précédentes, caractérisée en ce que le produit pharmaceutique contient la 2',4',6'-triméthoxy-4-(1-pyrrolidinyl)butyrophénone, les sels correspondants et/ou les dérivés de ce composé en une concentration qui varie de 1% en poids à 50% en poids.

7. Utilisation suivant l'une quelconque des revendications précédentes, caractérisée en ce que le produit pharmaceutique contient de la p-déméthyl-2',4',6'-triméthoxy-4-(1-pyrrolidinyl)butyrophénone.
